(19) Europäisches Patentamt
European Patent Office
Office européen des brevets

(11) EP 0 719 279 B1

(12) **EUROPÄISCHE PATENTSCHRIFT**

(45) Veröffentlichungstag und Bekanntmachung des Hinweises auf die Patenterteilung:
**19.11.1997 Patentblatt 1997/47**

(21) Anmeldenummer: **94924885.0**

(22) Anmeldetag: **19.08.1994**

(51) Int Cl.$^6$: **C07K 5/02**

(86) Internationale Anmeldenummer:
**PCT/EP94/02760**

(87) Internationale Veröffentlichungsnummer:
**WO 95/07928 (23.03.1995 Gazette 1995/13)**

# (54) VERFAHREN ZUR REINIGUNG VON 1- N(2 -((S)-ETHOXYCARBONYL)-3-PHENYLPROPYL)-N6 -TRIFLUORACEYTL]-L-LYSYL-L-PROLIN (LISINOPRIL(TFA)ETHYLESTER)

PROCESS FOR PURIFYING 1- N(2 -((S)-ETHOXYCARBONYL)-3-PHENYLPROPYL)-N6 -TRIFLUOROACETYL]-L-LYSYL-L-PROLIN (LISINOPRIL(TFA)ETHYLESTER)

PROCEDE DE PURIFICATION DE 1- N(2 -((S)-ETHOXYCARBONYLE)-3-PHENYLPROPYLE)-N6 -TRIFLUOROACETYLE-L-LYSYLE-L-PROLINE (LISINOPRIL(TFA)ESTER D'ETHYLE)

(84) Benannte Vertragsstaaten:
**AT BE CH DE DK ES FR GB GR IE IT LI NL PT SE**

(30) Priorität: **17.09.1993 DE 4331540**

(43) Veröffentlichungstag der Anmeldung:
**03.07.1996 Patentblatt 1996/27**

(73) Patentinhaber: **Degussa Aktiengesellschaft**
**60311 Frankturt (DE)**

(72) Erfinder:
- **KOTTENHAHN, Matthias**
  **D-63579 Freigericht (DE)**
- **DRAUZ, Karlheinz**
  **D-63579 Freigericht (DE)**

(56) Entgegenhaltungen:
**EP-A- 0 336 368**
**EP-A- 0 523 449**
**WO-A-94/15957**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 719 279 B1

Printed by Jouve, 75001 PARIS (FR)

## Beschreibung

Die Erfindung betrifft ein Verfahren zur Reinigung von 1-[$N^2$-((S)-Ethoxycarbonyl)-3-phenylpropyl)-$N^6$-trifluoracetyl]-L-lysyl-L-prolin (Verbindung I) durch Extraktion und Kristallisation.

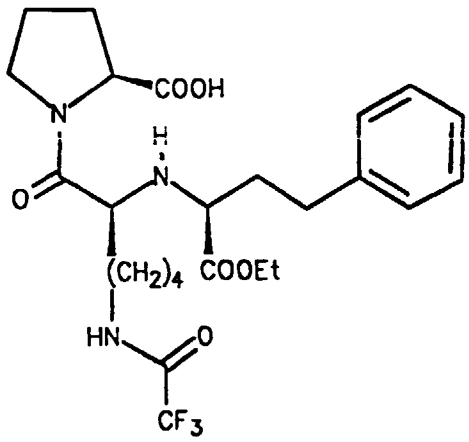

(I).

N-substituierte Aminosäuren dieses Typs sind wertvolle Zwischenprodukte für die Herstellung von Inhibitoren des Angiotensin Converting Enzymes (ACE), die als Blutdruckregulatoren wirken. I ist das direkte Vorprodukt für 1-[$N^2$-((S)-Carboxy)-3-phenylpropyl)]-L-lysyl-L-prolin (Lisinopril) Verbindung II, welches hervorragende Therapieresultate bei der Bluthochdruckbekämpfung aufweist (Zerstril®, Coric®, Prinivil®).

Verbindung I erhält man nach dem Stand der Technik durch reduktive Aminierung von 2-Oxo-4-phenylbuttersäureethylester mit dem Dipeptid Lys(Tfa)-Pro.

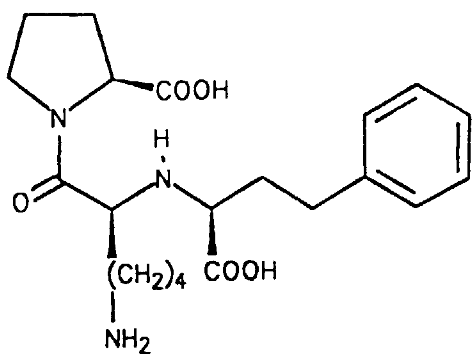

(II)

Im J. Org. Chem. 1988, 53, 836 - 844 wird ein solches Verfahren beschrieben. I wird danach in einer Ausbeute von 42 % durch basische Extraktion der Reaktionsrohlösung, eine nachgeschaltete Extraktion des Produktes in ein organisches Lösungsmittel bei pH 4,6 und anschließende Kristallisation aus Methyl-tert.Butylether, Cyclohexan erhalten.

Die DE-OS 41 23 248 betrifft die Synthese von I, das gemäß Beispiel 3 mit einer Ausbeute von 60 % gewonnen wird. Die Aufarbeitung der nach diesem Verfahren erhaltenen Reaktionsrohlösung beinhaltet neben einem basischen und einem sauren Extraktionsschritt eine Kristallisation aus Methyl-tert.Butylether. Grundsätzlich sind auch weitere, nicht auf reduktiver Aminierung beruhende, jedoch weniger vorteilhafte Verfahren zur Herstellung von Verbindung I bekannt (EP 0 336 368 A2).

Die basische Extraktion - eine Extraktion der wässrigen Produktphase ist notwendig, um Verunreinigungen aus der Aminierungsreaktion zu entfernen - ist jedoch stets mit Produktverlusten durch Amid- und/oder Esterspaltung von I nach II, bzw. von I zu Verbindungen III und IV verbunden.

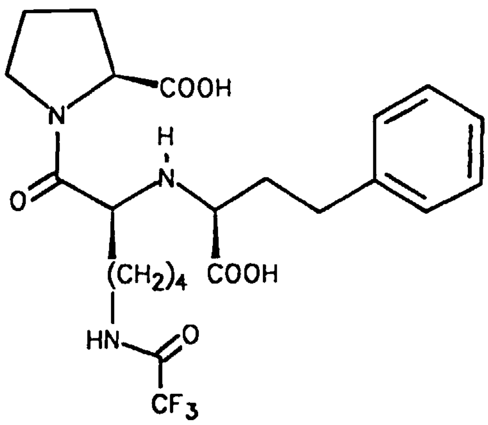

(III)

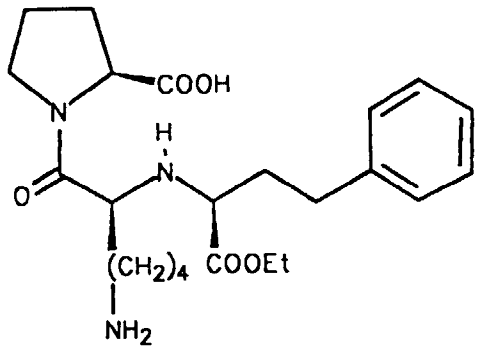

(IV)

Um diese Produktverluste zu minimieren, muß bei mit tiefen Temperaturen exakt eingestellten pH-Werten und möglichst kurzen Kontaktzeiten gearbeitet werden. Dies ist aufwendig und insbesondere im technischen Maßstab mit Schwierigkeiten verbunden.

Die Kristallisation aus Methyl-tert.Butylether ist deshalb mit hohen Ausbeuteverlusten verbunden, da man, um die diastereomerenreine Verbindung (I) direkt erhalten zu können, in großer Verdünnung kristallisieren muß, was die Ausbeute schmälert. Läßt man (I) aus Lösungen mit hoher Konzentration kristallisieren, wird eine zusätzliche Umkristallisation notwendig. Beschrieben ist auch der Zusatz von Cyclohexan (J. Org. Chem, 1988, 53, 836 - 844) bei der Kristallisation zur Ausbeuteerhöhung. Dabei besteht jedoch die Gefahr der Abscheidung als Öl, was eine Isolierung des Produkts nicht nur im technischen Maßstab stark erschwert.

Aufgabe der Erfindung ist ein verbessertes Verfahren zur Reinigung von (I).

Diese Aufgabe wird erfindungsgemäß dadurch gelöst, daß man das insbesondere nach der reduktiven Aminierung durch Eindampfen der Reaktionslösung erhaltene Rohprodukt von (I) mit einem zweiphasigen System aus Wasser-Lösungsmittel gegebenenfalls mehrfach extrahiert, dessen Wasserphase auf einen pH-Wert zwischen 0 und 3,5, insbesondere 0,5 und 2, eingestellt wurde, nach dem Abtrennen der organischen Phase die verbleibende wässrige Lösung mit einer alkalisch wirkenden Verbindung, insbesondere NaOH oder KOH, auf einen pH-Wert zwischen 3,5 und 6,3, insbesondere 3,9 und 4,8, einstellt, anschließend mit einem organischen, mit Wasser nicht mischbaren Lösungsmittel oder Lösungsmittelgemisch gegebenenfalls mehrfach extrahiert, aus der organischen Phase (I) durch Eindampfen isoliert und nach dem Lösen in einem Gemisch aus Methyl-tert.Butylether und Methylcyclohexan durch Abkühlen auf Temperaturen auf -40°C bis +50°C. insbesondere auf -10°C bis 30°C, das Produkt (I) auskristallisieren läßt.

Das kristalline Produkt wird dann mit den bekannten Methoden abgetrennt.

Die saure Extraktion der wässrigen Produktlösung führt überraschenderweise nur zu kaum merklichen Produktverlusten bei gleichzeitig hoher Aufreinigung der wässrigen Produktphase und dies, obwohl die Trifluoracetylamidfunktion auch im sauren Medium abspaltbar ist. Die Lösung erweist sich unerwarteterweise auch als über längere Zeiträume (~ 48 h) im sauren stabil, was eine große technische Vereinfachung darstellt. Ein vorteilhafter pH-Bereich der sauren Extraktion liegt zwischen 0 und 3,5, besonders vorteilhaft ist ein Bereich zwischen 0,5 und 2. Die Extraktion kann bei Temperaturen zwischen 0°C und 60°C, besonders vorteilhaft zwischen 10°C und 40°C, insbesondere bei Raumtemperatur durchgeführt werden. Als Extraktionslösungsmittel können mit Wasser nicht mischbare Lösungsmittel, wie z. B. Toluol, Methyl-tert.Butylether, Ethylacetat, Isopropylacetat, Methylenchlorid, Trichlorethan, Chloroform oder deren Mischungen, eingesetzt werden.

Die oben beschriebenen Temperaturbereiche und Lösungsmittel / Lösungsmittelgemische gelten auch für den 2. Extraktionsschritt.

Die Umkristallisation des Produktes (I) erfolgt nach dem erfindungsgemäßen Verfahren aus einer Mischung von Methyl-tert.Butylether und Methylcyclohexan bei erniedrigten Temperaturen. Besonders vorteilhaft ist eine Vorkristallisation bei den unten angegebenen Temperaturen aus Methyl-tert.Butylether mit anschließendem Zusatz von Methylcyclohexan zur Komplettierung der Kristallisation. Man erhält durch den Zusatz von Methylcyclohexan deutlich reinere Produkte bei deutlich verminderter Neigung zur Abscheidung des Produktes als Öl als es nach Stand der Technik möglich war. Das Volumen-Verhältnis von Methyl-tert.Butylether zu Methylcyclohexan variiert dabei von 1:1 bis 20:1, besonders vorteilhaft ist ein Verhältnis von 2:1 bis 10:1. Die Kristallisation erfolgt bei Temperaturen zwischen -40°C und 50°C, besonders vorteilhaft zwischen -10°C und 30°C.

Der Konzentrationsbereich, ausgedrückt als Verhältnis von g Produkt (I) zu ml Lösungsmittel (einzeln (Methyl-tert. Butylether) oder gemeinsam (mit Methylcyclohexan), beläuft sich auf 1:0,5 bis 1:30, insbesondere 1:1 bis 1:10, dies natürlich in Abhängigkeit von den durch die Temperatur vorgegebenen Löslichkeiten.

Durch die nachfolgenden Beispiele soll das erfindungsgemäße Verfahren näher erläutert werden:

Beispiel 1:

1-[$N^2$-((S)-Ethoxycarbonyl)-3-phenylpropyl)-$N^6$-trifluoracetyl]-L-lysyl-L-prolin (Verbindung I)

Es wird eine reduktive Aminierung gemäß Patentanmeldung P 41 23 248.8 analog Beispiel 3 Seite 10 durchgeführt.
Aufarbeitung:
Die Reaktionslösung eines 150 mmol Ansatzes wurde im Vakuum bei 45°C Badtemperatur weitgehend eingedampft. Der Rückstand wurde in 750 ml Wasser aufgenommen und im Vakuum kurz angestrippt. Nach Zusatz von 150 ml 1,1,1-Trichlorethan wurde der pH-Wert mit konz. HCl auf 1 eingestellt. Man rührte 10 min nach und trennte anschließend die Phasen. Bei 5°C wurde nun in der wässrigen Phase ein pH-Wert von 4,6 eingestellt und diese 2x mit 300 ml 1,1,1-Trichlorethan extrahiert. Die organische Phase wurde im Vakuum eingedampft und in 250 ml Methyl-tert.Butylether aufgenommen. Durch Abkühlen auf 5°C und Animpfen entstand ein dicker Kristallbrei, der nach 5 Stunden unter starkem Rühren mit 65 ml Methylcyclohexan versetzt wurde. Nach weiteren 2 h bei 5°C wurde abgesaugt und im Vakuum getrocknet.
Ausbeute: 54 g (67.5 % der Theorie), Gehalt an SSS-Diastereomer: >99 %, $[\alpha]_D^{20}$: -25,6° [(c=1 in MeOH/ HCl (1:1)]

Beispiel 2:

1-[$N^2$-((S)-Ethoxycarbonyl)-3-phenylpropyl)-$N^6$-trifluoracetyl]-L-lysyl-L-prolin (Verbindung I)

Es wird eine reduktive Aminierung gemäß Patentanmeldung P 41 23 248.8 analog Beispiel 3 Seite 10 durchgeführt.
Aufarbeitung:
Die Reaktionslösung eines 150 mmol Ansatzes wurde im Vakuum bei 45°C Badtemperatur weitgehend eingedampft. Der Rückstand wurde in 750 ml Wasser aufgenommen und im Vakuum kurz angestrippt. Nach Zusatz von 120 ml Toluol und 30 ml Ethylacetat wurde der pH-Wert mit konz. HCl auf 1 eingestellt. Man rührte 10 min nach und trennte anschließend die Phasen. Bei 5°C wurde nun in der wässrigen Phase ein pH-Wert von 4.6 eingestellt und diese 1x mit 400 ml Ethylacetat extrahiert. Die organische Phase wurde im Vakuum eingedampft und in 250 ml Methyl-tert. Butylether aufgenommen. Durch Abkühlen auf 5°C und Animpfen entstand ein dicker Kristallbrei, der nach 5 Stunden unter starkem Rühren mit 65 ml Methylcyclohexan versetzt wurde. Nach weiteren 2 h bei 5°C wurde abgesaugt und im Vakuum getrocknet.
Ausbeute: 54,4 g (68 % der Theorie), Gehalt an SSS-Diastereomer: >99 %, $[\alpha]_D^{20}$: 25,5° [(c=1 in MeOH/ HCl (1:1)]

**Patentansprüche**

1. Verfahren zur Reinigung des insbesondere durch reduktive Aminierung erhaltenen 1-[$N^2$-((S)-Ethoxycarbonyl)-3-phenylpropyl)-$N^6$-trifluoracetyl]-L-lysyl-L-prolin (I)-Rohprodukts durch zwei Extraktionsschritte mit einem organischen Lösungsmittel und Kristallisation aus Methyl-tert.Butylether, **dadurch gekennzeichnet**, daß man das Rohprodukt im ersten Extraktionsschritt mit einem zweiphasigen System aus Wasser-Lösungsmittel behandelt, dessen Wasserphase auf einen pH-Wert zwischen 0 und 3,5 eingestellt wurde und im zweiten Extraktionsschritt die verbleibende wässrige Phase mit einer alkalisch wirkenden Verbindung auf einen pH-Wert zwischen 3,5 und 6,3 einstellt und mit einem organischen, mit Wasser nicht mischbaren Lösungsmittel bzw. Lösungsmittelgemisch behandelt und die Kristallisation unter Zusatz von Methylcyclohexan durchführt.

2. Verfahren gemäß Anspruch 1,
**dadurch gekennzeichnet**,
daß man den ersten Extraktionsschritt in einem pH-Wert-Bereich von 0,5 bis 2 durchführt.

3. Verfahren nach den Ansprüchen 1 und 2,
**dadurch gekennzeichnet**,
daß man als org. Lösungsmittel ein Gemisch aus Toluol und Ethylacetat einsetzt.

4. Verfahren gemäß einem oder mehreren der Ansprüche 1 bis 3,

**dadurch gekennzeichnet**,
daß man (I) zunächst aus Methyl-tert.Butylether vorkristallisieren läßt und anschließend durch Zusatz von Methylcylohexan die Kristallisation vervollständigt.

5. Verfahren nach einem oder mehreren der Ansprüche 1 bis 4,
**dadurch gekennzeichnet**,
daß das Volumen-Verhältnis zwischen Methyl- tert.Butylether und Methylcyclohexan zwischen 1:1 und 20:1 liegt.

6. Verfahren nach Anspruch 5,
**dadurch gekennzeichnet**,
daß das Volumen-Verhältnis zwischen Methyl- tert.Butylether und Methylcyclohexan zwischen 2:1 und 10:1 liegt.

7. Verfahren nach einem oder mehreren der Ansprüche 1 bis 6,
**dadurch gekennzeichnet,**
daß man in einem Temperaturbereich zwischen -40°C und 50°C kristallisieren läßt.

8. Verfahren nach Anspruch 7,
**dadurch gekennzeichnet,**
daß man in einem Temperaturbereich zwischen -10°C und 30°C kristallisieren läßt.

**Claims**

1. A process for purifying the crude product of 1-[$N^2$-((S)-ethoxycarbonyl)-3-phenylpropyl)-$N^6$-trifluoroacetyl]-L-lysyl-L-proline (I), in particular when obtained by reductive amination, by two extraction steps using an organic solvent and crystallisation from methyl-tert.butyl ether,
characterised in that
in the first extraction step the crude product is treated with a two-phase system comprising water and a solvent, the aqueous phase having a pH adjusted to between 0 and 3.5, and that in the second extraction step the retained aqueous phase is adjusted to a pH between 3.5 and 6.3 using an alkaline compound and is treated with an organic solvent or solvent mixture which is not miscible with water, and crystallisation is performed with the addition of methylcyclohexane.

2. A process according to Claim 1,
characterised in that the first extraction step is performed at a pH between 0.5 and 2.

3. A process according to Claims 1 and 2,
characterised in that
the organic solvent used is a mixture of toluene and ethyl acetate.

4. A process according to one or more of Claims 1 to 3,
characterised in that
(I) is initially allowed to pre-crystallise from methyl-tert.butyl ether and the crystallisation procedure is subsequently completed by adding methylcyclohexane.

5. A process according to one or more of Claims 1 to 4,
characterised in that
the ratio by volume of methyl-tert.butyl ether to methylcyclohexane is between 1:1 and 20:1.

6. A process according to Claim 5,
characterised in that
the ratio by volume of methyl-tert.butyl ether to methylcyclohexane is between 2:1 and 10:1.

7. A process according to one or more of Claims 1 to 6,
characterised in that
crystallisation takes place at a temperature between -40°C and 50°C.

8. A process according to Claim 7,

characterised in that
crystallisation takes place at a temperature between -10°C and 30°C.

## Revendications

1. Procédé de purification du produit brut de 1-[$N^2$-((S)-éthoxycarbonyle)-3-phénylpropyle)-$N^6$-trifluoroacétyle]-L-Lysyle-L-proline (I) obtenu en particulier par amination réductrice par deux étapes d'extraction avec un solvant organique et cristallisation de l'éther méthylterbutylique,
caractérisé en ce qu'
on traite le produit brut dans la première étape d'extraction avec un système à deux phases à base d'eau, solvant, dont la phase aqueuse a été ajustée à une valeur de pH comprise entre 0 et 3,5 et en ce qu'on ajuste dans une deuxième étape d'extraction la phase aqueuse qui demeure, à une valeur de pH comprise entre 3,5 et 6,3 avec un composé à réaction alcaline et en ce qu'on traite avec un solvant ou un mélange de solvants organiques, non miscibles à l'eau et que l'on effectue la cristallisation en ajoutant du méthylcyclohexane.

2. Procédé conformément à la revendication 1,
caractérisé en ce qu'
on exécute la première étape d'extraction dans une plage de valeurs de pH allant de 0,5 à 2.

3. Procédé selon les revendications 1 et 2,
caractérisé en ce qu'
on met en oeuvre comme solvant organique un mélange de toluène et d'acétate d'éthyle.

4. Procédé conformément à une ou plusieurs des revendications 1 à 3,
caractérisé en ce qu'
on fait en premier lieu pré-cristalliser de l'éther méthylterbutylique et ensuite par addition de méthylcyclohexane, on rend complète la cristallisation.

5. Procédé selon une ou plusieurs des revendications 1 à 4,
caractérisé en ce que
le rapport en volume entre l'éther méthylterbutylique et le méthylcyclohexane se situe entre 1:1 et 20:1.

6. Procédé selon la revendications 6,
caractérisé en ce que
le rapport en volume entre l'éther méthylterbutylique et le méthylcyclohexane se situe entre 2:1 et 10:1.

7. Procédé selon l'une ou plusieurs des revendications 1 à 6,
caractérisé en ce qu'
on fait cristalliser dans une plage de températures comprise entre -40°C et 50°C.

8. Procédé selon la revendication 7,
caractérisé en ce qu'
on fait cristalliser dans une plage de températures comprise entre -10°C et 30°C.